# EUROPÄISCHE PATENTSCHRIFT

(11) **EP 2 750 751 B1**
(45) Veröffentlichungstag und Bekanntmachung des Hinweises auf die Patenterteilung: **13.04.2022**
(21) Anmeldenummer: 12761899.9
(22) Anmeldetag: 31.08.2012
(51) Int. Cl.: A61M 25/00, A61M 25/01, A61M 1/00, A61B 17/22

(54) **ASPIRATIONSKATHETER**
ASPIRATION CATHETER
CATHÉTER D'ASPIRATION

(30) Priorität: 31.08.2011 DE 102011111534
(43) Veröffentlichungstag der Anmeldung: 09.07.2014
(73) Patentinhaber: QUALIMED Innovative Medizinprodukte Gesellschaft mit beschränkter Haftung, 21423 Winsen/Luhe (DE)
(72) Erfinder: GÜLCHER, Manfred, 46348 Raesfeld-Erle (DE); SCHMITT, Martina, Maria, 21441 Garstedt (DE)
(74) Vertreter: Thiel, Christian
(86) Internationale Anmeldenummer: PCT/EP2012/003656
(87) Internationale Veröffentlichungsnummer: WO 2013/029795

(56) Entgegenhaltungen:
- EP-A1- 1 681 075
- WO-A1-2010/036541
- US-A- 5 456 680
- US-A1- 2009 018 502

## Beschreibung

Aspirationskatheter zur Extraktion von thrombotischen Material aus Blutgefäßen mit einem ersten und einem zweiten Lumen, wobei das erste Lumen distal eine Aspirationsöffnung aufweist und das zweite Lumen, das einen kleineren Querschnitt hat als das erste Lumen und koaxial im distalen Bereich des ersten Lumens verläuft, eine laterale sowie eine distale Öffnung aufweist, wobei die laterale Öffnung durch die Katheterwandung reicht.

In menschlichen Blutgefäßen, vor allem im koronaren Bereich, kommt es immer wieder zur Ausbildung von Ablagerungen, etwa Plaques oder Thromben, die den Blutdurchfluss behindern oder gänzlich unterbinden. Im Falle einer solchen Blockade in einem Versorgungsgefäß im koronaren Bereich kann es zu schweren Komplikationen kommen, etwa Infarkten, gesundheitlichen Folgeschäden bis hin zum Tod. Bei der ärztlichen Versorgung solcher Patienten ist deshalb eine schnelle Beseitigung solcher Engstellen oder Blockaden erforderlich.

Bei der Behandlung sklerotischer Ablagerungen in Gefäßsystemen werden häufig Techniken angewandt, die zur Aufweitung des Gefäßes führen (Angioplastie), etwa durch den Einsatz von Ballonkathetern. Häufig sind diese Techniken mit der Einpflanzung von Stents verbunden. Im koronaren Bereich werden Engpässe oder Blockaden häufig auch durch Bypässe beseitigt.

Im Falle der Ausbildung von Thromben oder der Ablagerungen von Emboli werden häufig Aspirationskathether eingesetzt, mit denen das thrombotische oder embolische Material aus dem Blutgefäß abgesaugt wird. Solche Aspirationskatheter haben im distalen Bereich eine Öffnung, durch die Blut in den Katheterraum eintreten kann. Am proximalen Ende wird eine herkömmliche Kolbenspritze angeschlossen. Mit Hilfe der Spritze wird ein Vakuum erzeugt und Blut mit thrombotischem Material durch den Katheter hindurch abgesaugt.

Aspirationskatheter werden auf übliche Art und Weise mit Hilfe eines Führungsdrahts platziert. Hierzu wurden zwei individuelle Techniken entwickelt, die sich an und für sich bewährt haben.

Bei der "Over-the-Wire"-Technik wird der Aspirationskatheter auf einen bereits gelegten Führungsdraht geschoben und entlang diesem Führungsdraht zum Einsatzort transportiert.

Bei der "Rapid Exchange"-Technik weist der Aspirationskatheter im distalen Bereich ein zusätzliches Katheterrohr auf, das auf einen bereits gelegten Führungsdraht aufgeschoben werden kann, so dass der Aspirationskatheter auf diesem Führungsrohr entlang dem Führungsdraht zum Einsatzort geschoben werden kann.

Bei der "Over-the-Wire"-Technik liegt das Problem darin, dass der Führungsdraht im Inneren des Aspirationskatheters durch seine Reibungskräfte den Kathetervorschub an den Einsatzort behindern kann. Zudem verengt der Führungsdraht das Sauglumen über die gesamte Katheterlänge und beschränkt damit die Saugwirkung.

Bei der "Rapid Exchange"-Technik ist das zusätzliche Lumen außerhalb des Aspirationskathethers platzraubend und erschwert die Manövrierung.

Bei einer Weiterentwicklung des "Rapid Exchange"-Katheters ist im distalen Bereich des Aspirationskatheters ein zusätzliches Lumen für den Führungsdraht vorhanden. Dieses Lumen hat eine distale Öffnung sowie eine laterale Öffnung, so dass der Aspirationskatheter bei der Platzierung mit seinen distalen Ende auf den Führungsdraht aufgeschoben werden kann, der dann aus der lateralen Öffnung austritt und parallel zum Aspirationskatheter verläuft. Mit dieser Variante lässt sich ein Aspirationskatheter schnell und zielgenau platzieren. Andererseits birgt diese Variante die Gefahr, dass der Aspirationskatheter bei der Platzierung an seiner Schwachstelle, dem seitlichen Austritt des zweiten Lumens, abknickt. In einem solchen Fall ist die Saugleistung des Aspirationskatheters im günstigsten Fall beeinträchtigt; in der Regel ist ein Austausch des Aspirationskatheters erforderlich.

Aspirationskatheter entfalten ihre volle Wirksamkeit dann, wenn es gelingt, die distale Katheterspitze durch eine Engstelle - in der Regel einen Thrombus - hindurch zu manövrieren und die Saugwirkung erst anschließend, beim Zurückziehen des Aspirationskatheters, zur Entfaltung kommen zu lassen. Die Gestaltung der Katheterspitze spielt hier ebenso eine Rolle wie die Anordnung der Aspirationsöffnung, die eine seitliche Ausrichtungskomponente haben sollte. Diese Anforderungen müssen mit der Anordnung des Lumens für den Führungsdraht in Einklang gebracht werden.

Aus der EP 1 681 075 A1 geht ein Aspirationskatheter gemäß Oberbegriff des Anspruchs 1 hervor. Die US 2009/018502 A1 beschreibt einen Ballonkatheter, der von einem Over-the-Wire-Status in einem Rapid-Exchange-Status überführt werden kann und über einen Stabilisierungsdraht verfügt. Die US 5,456,680 offenbart einen Katheter mit einer Faseroptik, der in einem zentralen Lumen einen Führungsdraht aufweist, der auch der Stabilisierung dient. Aus der WO 2010/036541 A1 geht ein Katheter mit einem Führungsdraht, der in einem separaten Lumen geführt wird und gegen einen anderen Führungsdraht definierter Steifheit ausgetauscht werden kann.

Aufgabe der Erfindung ist es, einen Aspirationskatheter bereitzustellen, der die vorstehend geschilderten Nachteile bekannter Aspirationskatheter, darunter insbesondere die Knickneigung von Aspirationskathetern nach dem "Rapid Exchange"-System vermeidet.

Der erfindungsgemäße Aspirationskatheter stellt eine Kombination zweier bekannter Prinzipien dar, des "Over-the-Wire"-Prinzips und des "Rapid Exchange"-Prinzips. Ein erstes weiteres Lumen dient der Aspiration von thrombotischem Material aus dem jeweils betroffenen Blutgefäß, ein engeres innerhalb des ersten Lumens angeordnetes zweites Lumen verläuft koaxial im distalen Bereich des ersten Lumens und dient der Führung am Führungsdraht.

Das koaxial im distalen Bereich des ersten Lumens angeordnete zweite Lumen weist eine distale und laterale Öffnung auf dergestalt, dass der Aspirationskatheter an einem durch das zweite Lumen geführten Führungsdraht entlang an seinen Einsatzort geführt werden kann, quasi auf dem Führungsdraht "reiten" kann. Die laterale Öffnung reicht durch die Katheterwandung des ersten Lumens, die distale Öffnung des zweiten Lumens endet distal von der Aspirationsöffnung des ersten Lumens, bildet insbesondere auch die distale Spitze des Aspirationskatheters insgesamt. Die laterale Öffnung befindet sich proximal von der Aspirationsöffnung des ersten Lumens, wobei der Abstand zur Aspirationsöffnung und zur Katheterspitze so bemessen ist, dass eine präzise Führung des Aspirationskatheters am Führungsdraht möglich ist. Es ist zweckmäßig, die Wandung des zweiten Lumens an der Innenwandung des Katheters (des ersten Lumens) festzulegen.

Erfindungsgemäß befindet sich innerhalb des ersten Lumens ein Stabilisierungsdraht, der dort verschiebbar gelagert bzw. angeordnet ist. Bei diesem Stabilisierungsdraht handelt es sich im Prinzip um einen üblichen Führungsdraht, der jedoch nicht dazu dient, den Katheter zu führen, sondern dem Katheter bei der Führung entlang des Führungsdrahts so viel Stabilität verleihen soll, dass es bei Gefäßwindungen und -abzweigungen nicht zum Abknicken des Katheterschlauchs kommt. Dieser Stabilisierungsdraht reicht bis über die laterale Öffnung des zweiten Lumens hinaus und endet in jedem Fall vor der Aspirationsöffnung.

Vorzugsweise endet der Stabilisierungsdraht unmittelbar hinter der lateralen Öffnung des zweiten Lumens. Dies stellt sicher, dass der distale Bereich des Aspirationskatheters in seiner Flexibilität und Manövrierfähigkeit nicht noch zusätzlich durch den Stabilisierungsdraht eingeschränkt wird. Gleichzeitig ist aber die Reichweite über die laterale Öffnung des zweiten Lumens hinaus ausreichend, eine Knickgefahr in diesem sensiblen Bereich zu vermeiden.

Insbesondere weist der erfindungsgemäße Katheter vier einzelne Bereiche auf, die Zonen A (distal) bis D (proximal). Zone A, die von der Katheterspitze bis zum Port reicht, stellt den Aspirationsbereich dar, der eine gewisse Stabilität braucht, um unter einem reduzierten Druck nicht zu kollabieren. Zone B, die sich proximal an die Zone A anschließt, hat eine erhöhte Flexibität, die in kurvenreichen Gefäßen von Vorteil ist.

Zone C, proximal zur Zone B gelegen und von der Zone B durch einen Übergangspunkt getrennt, an dem die Materialien der Zonen B und C zusammengeführt sind, weist eine gute Lenkbarkeit auf, um den Katheter durch das Gefäßsystem eines Patienten zu führen. Zone D, der proximale Sektor, steht für eine gute Kraftübertragung, d.h. eine gute Übertragung der auf das proximale Katheterende angewandten Kraft auf die Katheterspitze.

Die einzelnen Abschnitte sind jeweils aus Materialien hergestellt, die die benötigten Eigenschaften aufweisen und an und für sich bekannt sind. Häufig verwandte Materialien sind Polyamide, etwa Grillamid, Peba und Mischungen davon, insbesondere für die Zonen B bis D wie auch für den Schlauch des inneren Lumens.

Die Länge der einzelnen Abschnitte kann, abhängig von den jeweiligen Anforderungen, innerhalb gewisser Bandbreiten variieren. Es ist zu berücksichtigen, dass eine Vergrößerung der Länge der Zone B auf Kosten der Zonen C und/oder D für eine verbesserte Flexibilität zu Lasten der Kraftübertragung und der Lenkbarkeit geht. Üblicherweise hat in einem erfindungsgemäßen Katheter die Zone A eine Länge von 20 bis 50 mm, die Zone B eine Länge von 200 bis 500 mm und die Zonen C und D eine Länge zusammen von bis zu 1250 mm.

Bei dem erfindungsgemäßen Aspirationskatheter ist es bevorzugt, dass das Rohr des zweiten Lumens über das distale Ende des ersten Lumens hinausragt und mit seiner distalen Öffnung das distale Ende des Aspirationskatheters bildet. Da das Rohr des zweiten Lumens einen erheblich kleineren äußeren Durchmesser hat als der Aspirationskatheter selbst, führt dies dazu, dass das distale Ende des Katheters nasenförmig vorspringt. Diese Ausbildung der Katheterspitze hat den Effekt, dass mit der "Nase" ein Thrombus oder Embolus in einem Blutgefäß durchstoßen werden kann, so dass die Aspirationsöffnung distal vom Gefäßverschluss zum Liegen kommt und die Aspirationswirkung beim Zurückziehen des Katheters angewandt werden kann.

Der Durchmesser des zweiten Lumens ist allgemein erheblich kleiner als der des ersten Lumens. Insbesondere ist das Querschnittsverhältnis von erstem Lumen zu zweitem Lumen im Bereich von 3:1 bis 10:1, vorzugsweise im Bereich von 4:1 bis 6:1.

Die anmeldungsgemäß gebrauchten Begriffe "distal" und "proximal" sind so zu verstehen, dass das in das Gefäß reichende Ende des Katheters, also das vom behandelnden Arzt wegweisende Ende des Katheters das distale Ende ist, während die zum Arzt weisende Seite als proximal bezeichnet wird. Bezugspunkt ist also der behandelnde Arzt bzw. die Eintrittsstelle des Katheters in den Körper eines Patienten, zumeist die Leiste.

Die Aspirationsöffnung des ersten Lumens bildet insbesondere zugleich auch das Ende des ersten Lumens. Erfindungsgemäss ist eine schräg oder leicht lateral versetzte Anordnung am distalen Ende des Katheterschlauchs, so dass eine Saugwirkung sowohl distal als auch lateral eintreten kann. Die Aspirationsöffnung hat insbesondere eine tropfenförmige oder ovale Form. Um ein Ansaugen der Aspirationsöffnung an einer Gefäßwand zu verhindern ist, in der Höhe der Aspirationsöffnung im ersten Lumen auf der in etwa gegenüberliegende Seite eine Druckausgleichsöffnung vorgesehen, die erheblich kleiner ausgebildet ist, so dass sie zwar einen Druckausgleich herbeiführen kann, wenn eine Verstopfung der Aspirationsöffnung eingetreten ist, jedoch die Saugwirkung der Aspirationsöffnung nicht nennenswert zu verkleinern vermag.

Diese Druckausgleichsöffnung ist seitlich gegenüber dem Verlauf des zweiten Lumens versetzt.

Um eine exakte Platzierung des Aspirationskatheters und insbesondere der Aspirationsöffnung des ersten Lumens zu ermöglichen, weist das Rohr des zweiten Lumens in Höhe der Aspirationsöffnung eine Markierung auf. Diese Markierung liegt in Form einer Banderole, Manschette oder eines Rings vor und ist vorzugsweise aus einer Platin-Iridium-Legierung 90/10 gefertigt, die gut röntgensichtbar ist.

Im Sinne einer guten Manövrierfähigkeit des Aspirationskatheters ist es zweckmäßig, den Stabilisierungsdraht hinsichtlich seiner Steifigkeit bzw. Flexibilität zu variieren. In der Regel ist der proximale Teil des Stabilisierungsdrahts weniger flexibel und steifer als der distale Teil. Die Steifigkeit kann dabei kontinuierlich von proximal nach distal abnehmen, aber auch in Sprüngen.

Der erfindungsgemäße Aspirationskatheter kann eine übliche hydrophile Beschichtung aufweisen, die sich aber in der Regel nicht über den gesamten Katheter erstreckt, sondern proximal von der lateralen Öffnung des zweiten Lumens endet.

Der Stabilisierungsdraht ist zweckmäßigerweise im distalen Bereich mit einer Markierung versehen. Diese Markierung dient dazu, dem behandelnden Arzt anzuzeigen, dass beim Zurückziehen des Stabilisierungsdrahts, um den Aspirationsweg freizugeben, das Ende des Stabilisierungsdrahts außerhalb des Aspirationswegs liegt. In der Regel ist die Aspirationsspritze über einen Y-Verbinder und ein Luerlock an den Katheter angeschlossen, so dass der Stabilisierungsdraht mit seinem Ende jenseits des Y-Abzweigs liegen sollte. Ein entsprechender Abstand zum distalen Ende des Stabilisierungsdrahts und eine ggf. vorhandene Vormarkierung sind sinnvoll.

Ferner kann es zweckmäßig sein, das distale Ende des Stabilisierungsdrahtes etwas zu verdicken, dergestalt, dass er nur mit erhöhtem Kraftaufwand völlig herausgezogen werden kann; ergänzend kann der Katheter im proximalen Bereich mit einer leichten Verengung versehen sein, um die Verdickung des Stabilisierungsdrahtes zu "fangen".

Die Markierung zeigt zugleich die im Katheter verbleibende Länge des Stabilisierungsdrahts an, die unschädlich für den Aspirationsvorgang sein muss.

Die Erfindung betrifft schließlich einen Aspirationskatheter der vorstehend beschriebenen Art im Set mit einem dimensionsmäßig darauf abgestimmten Führungsdraht und Führungskatheter sowie ggf. einer Spritze zum Absaugen von thrombotisches Material enthaltendem Blut.

Gemäß einer bevorzugten Ausführungsform weist der distale Bereich des ersten Lumens einen ovalen oder ellipsenförmigen Querschnitt auf, wobei die Aspirationsöffnung auf der Seite der Hauptachse des Ovals bzw. der Ellipse angeordnet ist. Der distale Bereich des erfindungsgemäßen Aspirationskatheters wird flach gedrückt, mit einer Aspirationsöffnung, die sich auf der flachen Seite gegenüber des zweiten Lumens befindet. Diese Gestaltung hat den Effekt, dass sich die Aspirationsöffnung weniger an die Gefäßwand anpasst und deshalb die Gefahr des Festsaugens geringer ist. Des Weiteren erlaubt die so breitere Aspirationsöffnung eine gründlichere Absaugung von thrombotischem Material, insbesondere auch in Plaqueform.

Es zeigen:
- Figur 1: eine Gesamtdarstellung des erfindungsgemäßen Aspirationskatheters;
- Figur 2a: den Spitzenbereich des Katheters C-C von Figur 1 in seitlicher Ansicht und
- Figur 2b: von unten;
- Figur 3a: den Katheter von Figur 1 an der Stelle A-A im Querschnitt und
- Figur 3b: den distalen Bereich des Katheters an der Stelle B-B von Figur 1 im Querschnitt;
- Figur 4: den Stabilisierungsdraht;
- Figur 5: den Endbereich des Katheters von Figur 1 im Detail sowie
- Figur 6: das Zusammenwirken von Katheter, Stabilisierungsdraht, Führungsdraht und zweitem Lumen und
- Figur 7: den anschnittsweisen Aufbau eines erfindungsgemäßen Katheters.

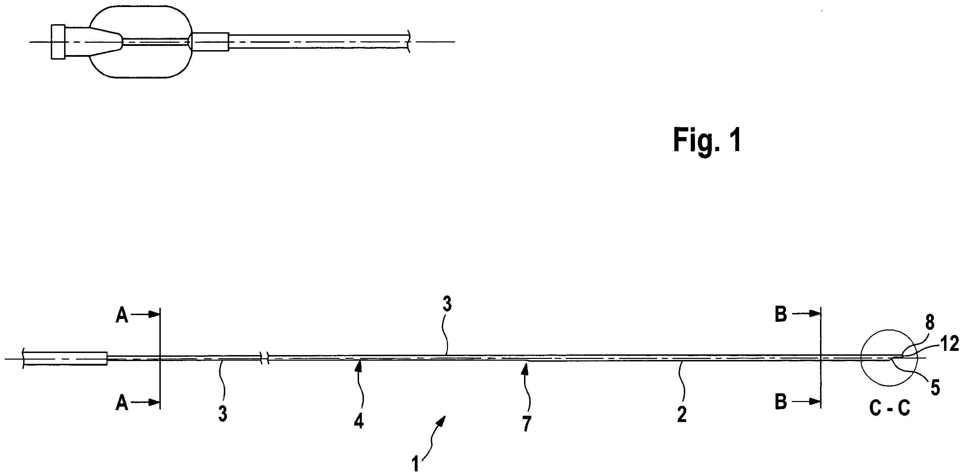

Figur 1 zeigt einen erfindungsgemäßen Aspirationskatheter 1 mit dem ersten Lumen 3, dem distalen Bereich 2 des ersten Lumens sowie der Aspirationsöffnung 5. Der Aspirationskatheter verfügt proximal über die übliche Ausrüstung zur Einführung durch einen konventionellen Y-Verbinder.

Der Katheter hat übliche Ausmaße, beispielsweise einen äußeren Durchmesser von 1,37 mm und einen inneren Durchmesser von 1,10 mm. Er wird aus üblichen Materialien gefertigt, etwa Peba, Polyamid oder Kombinationen derselben, wobei der Katheter selbst sektionsweise, je nach Anforderung an Steifigkeit und Flexibilität, aus verschiedenen Materialien bestehen kann. Beispielsweise ist der in Figur 1 dargestellte Katheter im proximalen Bereich bis zum Übergangspunkt 4 aus einem steiferen Material hergestellt und distal vom Übergangspunkt 4 aus einem weniger steifen Material. Dies verbessert die Einführbarkeit in ein Gefäßsystem. Distal vom Übergangspunkt 4 beginnt die Zone B, die bis zu Eintrittsöffnung 7 des zweiten Lumens 6 reicht und eine erhöhte Flexibilität aufweist.

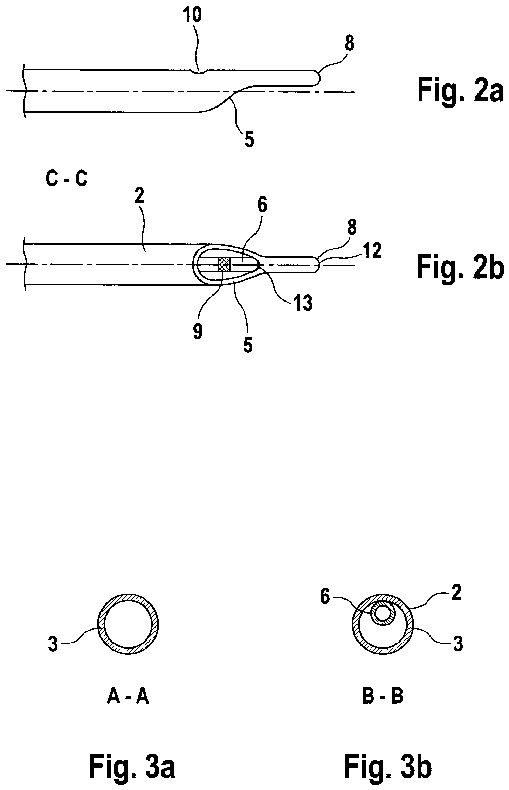

Der erfindungsgemäße Katheter weist einen distalen Bereich 2 auf, der an der Eintrittsöffnung 7 des zweiten Lumens 6 (Port) beginnt und bis zur Katheterspitze reicht. Der distale Bereich entspricht der Zone A und hat beispielsweise eine Länge von 30 mm. Innerhalb dieses distalen Bereichs 2 verläuft das zweite Lumen 6 (siehe Fig. 2) das in der distalen Öffnung 8, die mit der Katheterspitze 12 zusammenfällt, ausläuft. Unterhalb der Katheterspitze 12 und des zweiten Lumens 6, etwas nach proximal versetzt, befindet sich die Aspirationsöffnung 5.

Die Einheit C-C ist in Figur 2 dargestellt und zeigt die Katheterspitze von der Seite (a) und von unten (b). In der seitlichen Ansicht erscheint das Auslaufen des zweiten Lumens mit seiner Öffnung 8 als Nase, die die Katheterspitze 12 ausbildet. Unterhalb dieser Nase befindet sich die Aspirationsöffnung 5, gegenüberliegend ein Druckausgleichsloch 10, das nicht der Absaugung dient, sondern lediglich als Druckausgleich, insbesondere, um zu verhindern, dass sich die Aspirationsöffnung an einer Gefäßwand festsaugt.

Figur 2b zeigt die Einzelheit C-C aus Figur 1 von unten, also von der Seite der Aspirationsöffnung 5. Zu erkennen ist das distale Ende 12 des Katheters, das gleichzeitig das distale Ende und die distale Öffnung 8 des zweiten Lumens 6 ist. Der Verlauf des zweiten Lumens 6 wird von einem auf der gegenüberliegenden Seite des distalen Bereichs 2 verlaufendes Rohr gebildet. Das zweite Lumen 6 trägt eine Markermanschette 9 aus Pt-Ir 90/10, die den behandelnden Arzt als Orientierungshilfe dient.

Das zweite Lumen 6 verläuft an der der Aspirationsöffnung 5 gegenüberliegenden Seite des distalen Bereichs 2 des Katheters 3. Zu erkennen ist, dass der Durchmesser des zweiten Lumens 6 erheblich kleiner ist als der des ersten Lumens 3 oder des distalen Bereichs 2. Die vom distalen Ende 12 des Lumens 6 gebildete Nase reicht über das distale Ende 13 des ersten Lumens hinaus und dient dazu, einen bestehenden Thrombus mit Hilfe des Aspirationskatheters zunächst zu durchstoßen und dann unter Zurückziehung des Aspirationskatheters thrombotisches Material ab- und anzusaugen.

Figur 3a zeigt den Querschnitt des Aspirationskatheters 3 an der Stelle A-A. Der Katheter hat einen äußeren Durchmesser von in diesem Fall 1,37 mm und einen inneren Durchmesser von 1,10 mm und besteht aus einem flexiblem Rohr aus einem medizinisch zulässigen Kunststoffmaterial, etwa Pebax, Polyamid oder Teflon oder Mischungen davon.

Figur 3b zeigt den distalen Bereich 2 des erfindungsgemäßen Aspirationskatheters entlang der Schnittlinie B-B. Gezeigt ist das erste Lumen 3 des Aspirationskatheters sowie das zweite Lumen 6, das der Führung des Katheters über einen Rapid-exchange-Führungsdraht dient. Das zweite Lumen ist mit einem inneren Durchmesser von 0,42 mm und einem äußeren Durchmesser von 0,57 mm erheblich kleiner als das erste Lumen. Das Rohr des zweiten Lumens 6 ist zweckmäßigerweise an der Innenwand des Rohrs des ersten Lumens 3 festgelegt. Das Querschnittsverhältnis liegt damit bei etwa 5:1 (erstes Lumen : zweites Lumen).

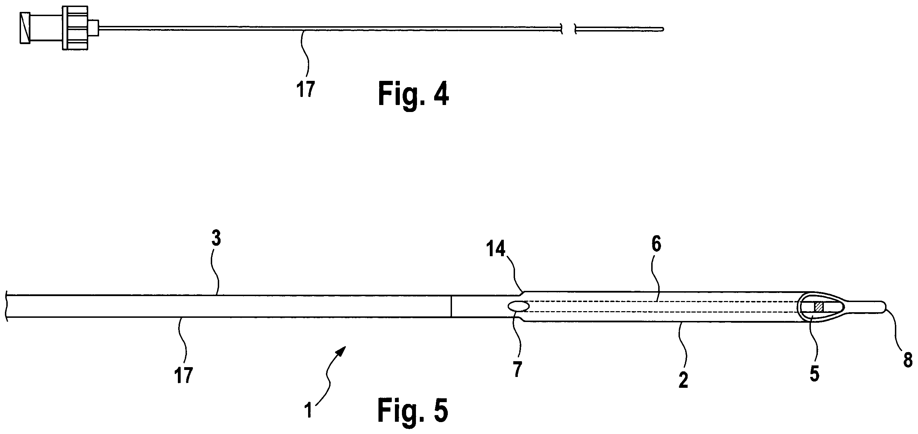

Figur 4 zeigt den Stabilisierungsdraht mit der Einführungshilfe, der bei einer Länge von beispielsweise 1.400 mm einen Durchmesser von 0,62 mm aufweist und aus einem herkömmlichen Federstahl gefertigt ist, etwa dem gleichen Material, wie es für einen herkömmlichen Führungsdraht verwandt wird.

Figur 5 zeigt den erfindungsgemäßem Aspirationskatheter 3 mit seinem distalen Bereich 2. Zu erkennen ist der Port 7 (laterale Öffnung) der das proximale Ende des gestrichelt dargestellten innenliegenden zweiten Lumens 6 darstellt, welches die Katheterspitze 12 ausbildet und in der distalen Öffnung 8 endet. Zu erkennen ist ferner die Aspirationsöffnung 5 mit der dahinterliegenden Markermanschette 9 des Lumens 2. Der Katheter weist proximal zum Port 7 eine hydrophile Beschichtung 17 auf.

Im distalen Bereich 2 weist der erfindungsgemäße Aspirationskatheter für das erste Lumen 3 vorzugsweise eine "platt gedrückte" Konfiguration auf, dergestalt, dass der Querschnitt in etwa oval oder ellipsenförmig verläuft. Dieser Verlauf setzt in etwa eine Höhe der lateralen Öffnung 7 des zweiten Lumens 6 ein und ist als abgeschwächte Stufe 14 dargestellt. Im distalen Bereich verfügt somit der Katheter 3 über eine längere Hauptachse quer zum Verlauf und eine kürzere Nebenachse senkrecht zur Hauptachse, hier in Blickrichtung. Der distale Bereich 2 bekommt damit etwa eine Schwertform. Die Aspirationsöffnung 5 befindet sich auf einer Breitseite des Schwerts.

Es versteht sich, dass die laterale Öffnung 7 des zweiten Lumens 6 auch auf der der Aspirationsöffnung 5 gegenüberliegenden Seite des distalen Bereichs 2 bzw. Katheters 3 angeordnet sein kann.

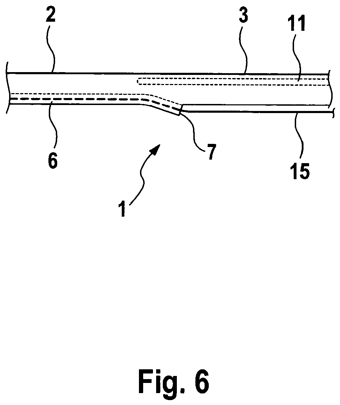

Figur 6 zeigt einen Ausschnitt aus einem erfindungsgemäßen Katheter 1 im Bereich des Übergangs in den distalen Abschnitt 2. Innerhalb des ersten Lumens 3 befindet sich das zweite Lumen 6 mit dem lateralen Port 7. Innerhalb des ersten Lumens 3 liegt der Stabilisierungsdraht 11, der distal vom Port 7 endet. Im Lumen 6 liegt ein Führungsdraht 15, der durch den Port 7 aus dem Katheter 1 austritt. Im Bereich des Ports 7 laufen Stabilisierungsdraht 11 und Führungsdraht 15 über eine kurze Strecke, etwa 5 bis 10 mm, parallel und erhöhen die Steifigkeit und damit die Knickfestigkeit des Katheters 1 an dieser knickempfindlichen Stelle.

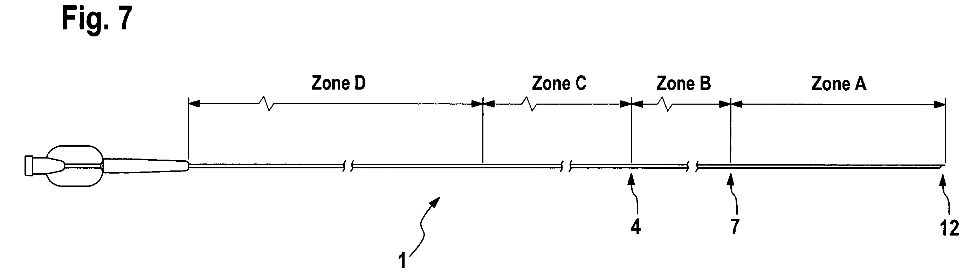

Figur 7 zeigt einen erfindungsgemäßen Aspirationskatheter 1 mit dem Übergangspunkt 4, dem Port 7 und der Katheterspitze 12 mit der vorstehend beschriebenen Zoneneinteilung.

Die Zone A stellt die distale Zone dar und hat eine Länge von etwa 30 mm. Zone A stellt den Aspirationsabschnitt dar, der sich durch eine erhöhte Druckfestigkeit auszeichnet.

Proximal an Zone A anschließend befindet sich Zone B, die eine erhöhte Flexibilität aufweist. Zone B reicht vom Übergangspunkt 4 bis zum Port 7. Die erhöhte Flexibilität von Zone B erleichtert die Führung in windungsreichen Gefäßen, etwa im Herzkranzbereich.

Proximal zu Zone B, anschließend an den Übergangspunkt 4 befindet sich die Zone C, die eine gute Lenkbarkeit aufweist, d.h. geeignet ist, Gefäßwindungen über eine längere Strecke problemlos zu folgen. Die proximal anschließende Zone D besteht aus einem Material, das eine gute Kraftübertragung bis hin zur Katheterspitze gewährleistet.

## Patentansprüche

1. Aspirationskatheter zur Extraktion von thrombotischem Material aus Blutgefäßen mit einem ersten (3) und einem zweiten Lumen (6), wobei das erste Lumen (3) distal eine Aspirationsöffnung (5) aufweist und das zweite Lumen (6), das einen kleineren Querschnitt hat als das erste Lumen (3) und achsparallel im distalen Bereich (2) des ersten Lumens (3) verläuft, eine laterale sowie eine distale Öffnung (8) aufweist, wobei die laterale Öffnung (7) durch die Katheterwandung reicht, wobei innerhalb des ersten Lumens (3) ein Stabilisierungsdraht (11) verschiebbar angeordnet ist, **dadurch gekennzeichnet, dass** der Stabilisierungsdraht (11) distal zur lateralen Öffnung (7) des zweiten Lumens (6), jedoch proximal zur Aspirationsöffnung (5) des ersten Lumens (3) endet, wobei die Aspirationsöffnung lateral oder schräg am distalen Ende (13) des ersten Lumens (3) angeordnet ist und das erste Lumen (3) auf der der Aspirationsöffnung (5) gegenüberliegenden Seite eine Druckausgleichsöffnung (10) aufweist.

2. Aspirationskatheter nach Anspruch 1, **dadurch gekennzeichnet, dass** der Stabilisierungsdraht (11) unmittelbar distal von der lateralen Öffnung (7) des zweiten Lumens endet.

3. Aspirationskatheter nach Anspruch 1 oder 2, **dadurch gekennzeichnet, dass** das zweite Lumen (6) über das distale Ende (13) des ersten Lumens (3) hinausragt und mit seiner distalen Öffnung (8) das distale Ende (12) des Aspirationskatheters (1) ausbildet.

4. Aspirationskatheter nach einem der Ansprüche 1 bis 3 mit einem distalen Ende (12), das nasenförmig ausgebildet ist und einen äußeren Querschnitt aufweist, der dem äußeren Querschnitt des zweiten Lumens (6) entspricht.

5. Aspirationskatheter nach Anspruch 5, **dadurch gekennzeichnet, dass** die Aspirationsöffnung (5) auf der dem zweiten Lumen (6) gegenüberliegenden Seite angeordnet ist.

6. Aspirationskatheter nach Anspruch 1, **dadurch gekennzeichnet, dass** die Druckausgleichsöffnung (10) gegenüber dem zweiten Lumen (6) seitlich versetzt ist.

7. Aspirationskatheter nach einem der vorstehenden Ansprüche, **gekennzeichnet durch** einen Markerring (9) am zweiten Lumen (6) in der Höhe der Aspirationsöffnung (5) des ersten Lumens (3).

8. Aspirationskatheter nach Anspruch 7 mit einem Markerring (9) aus Platin-Iridium-Legierung 90/10.

9. Aspirationskathether nach einem der vorstehenden Ansprüche, **dadurch gekennzeichnet, dass** der Stabilisierungsdraht (11) von proximal nach distal in seiner Flexibilität zunimmt.

10. Aspirationskathether nach einem der vorstehenden Ansprüche, **gekennzeichnet durch** eine äußere hydrophile Beschichtung (17).

11. Aspirationskatheter nach Anspruch 10, **dadurch gekennzeichnet, dass** die hydrophile Beschichtung (17) proximal zur lateralen Öffnung (7) des zweiten Lumens (6) endet.

12. Aspirationskatheter nach einem der vorstehenden Ansprüche, **dadurch gekennzeichnet, dass** der Stabilisierungsdraht (11) im Bereich seines distalen Endes mit einer Markierung versehen ist, die bei Herausziehen des Stabilisierungsdrahts (11) aus dem Katheter (1) die verbleibende Länge im Katheter (1) anzeigt.

13. Aspirationskatheter nach einem der vorstehenden Ansprüche, **dadurch gekennzeichnet, dass** der distale Bereich (2) des ersten Lumens (3) einen ovalen Querschnitt aufweist, wobei die Aspirationsöffnung (5) auf der Seite der Hauptachse des Ovals angeordnet ist.

14. Aspirationskatheter nach einem der Ansprüche 1 bis 13 mit dimensionsmäßig darauf abgestimmten Führungsdraht und Führungskathether.

15. Katheterset nach Anspruch 14 mit wenigstens einer Spritze zum Absaugen von thrombotisches Material enthaltendem Blut.

## Claims

1. Aspiration catheter for the extraction of thrombotic material from blood vessels, with a first lumen (3) and a second lumen (6), wherein the first lumen (3) is distally provided with an aspiration opening (5) and the second lumen (6) of smaller cross section than the first lumen (3) and extending coaxially in the distal region (2) of the first lumen (3) is provided with a lateral as well as distally located opening (8), said lateral opening (7) reaching through the wall of the catheter, a stabilization wire (11) being movably arranged within the first lumen (3) **characterized in that** the stabilization wire (11) terminates distally of the lateral opening (7) of the second lumen (6) but proximally of the aspiration opening (5) of the first lumen (3), the aspiration opening being arranged laterally or obliquely at the distal end (13) of the first lumen (3) and the first lumen (3) being provided with a pressure balancing opening (10) situated on the side opposite the aspiration opening (5).

2. Aspiration catheter according to claim 1, **characterized in that** the stabilization wire (11) terminates immediately distally of the lateral opening (7) of the second lumen.

3. Aspiration catheter according to claim 1 or 2, **characterized in that** the second lumen (6) projects beyond the distal end (13) of the first lumen (3) and with its distal opening (8) forms the distal end (12) of the aspiration catheter (1).

4. Aspiration catheter according to any one of the claims 1 to 3 having a distal end (12) designed so as to have a nose-like shape and an outer cross section that coincides with the outer cross section of the second lumen (6).

5. Aspiration catheter according to claim 1, **characterized in that** the aspiration opening (5) is arranged at the side opposite the second lumen (6).

6. Aspiration catheter according to claim 1, **characterized in that** the pressure balancing opening (10) is offset laterally with respect to the second lumen (6).

7. Aspiration catheter according to any one of the above claims, **characterized by** a marker ring (9) on the second lumen (6), said ring being arranged at the point where the aspiration opening (5) of the first lumen (3) is located.

8. Aspiration catheter according to claim 7 with a marker ring (9) made of platinum-iridium alloy 90/10.

9. Aspiration catheter according to any one of the above claims, **characterized in that** the flexibility of the stabilization wire (11) increases from proximal to distal.

10. Aspiration catheter according to any one of the above claims, **characterized by** an outer hydrophilic coating (17).

11. Aspiration catheter according to claim 10, **characterized in that** the hydrophilic coating (17) ends proximally to the lateral opening (7) of the second lumen (6).

12. Aspiration catheter according to any one of the above claims, **characterized in that** the stabilization wire (11) is provided with a marking in the area of its distal end, said marking indicates the length remaining in the catheter (1) when the stabilization wire (11) is extracted from the catheter (1).

13. Aspiration catheter according to any one of the above claims, **characterized in that** the distal area (2) of the first lumen (3) has an oval cross section, with the aspiration opening (5) being arranged on the side of the main axis of the oval.

14. Aspiration catheter according to any one of claims 1 to 13 with guidewire and guide catheter suitably sized for the relevant purpose.

15. Catheter set according to claim 13 with at least one syringe for the aspiration of blood containing thrombotic material.

## Revendications

1. Cathéter d'aspiration pour l'extraction de thrombus hors de vaisseaux sanguins avec une première (3) et une deuxième lumière (6), dans lequel la première lumière (3) présente distalement un orifice d'aspiration (5) et la deuxième lumière (6), qui a une section transversale plus petite que la première lumière (3) et s'étend de manière axialement parallèle dans la zone distale (2) de la première lumière (3) présente un orifice latéral ainsi qu'un orifice distal (8), dans lequel l'orifice latéral (7) traverse la paroi de cathéter, dans lequel un fil de stabilisation (11) est disposé de manière à pouvoir être déplacé à l'intérieur de la première lumière (3), **caractérisé en ce que** le fil de stabilisation (11) se termine sur le côté distal par rapport à l'orifice latéral (7) de la deuxième lumière (6), toutefois sur le côté proximal par rapport à l'orifice d'aspiration (5) de la première lumière (3), dans lequel l'orifice d'aspiration est disposé de manière latérale ou à l'oblique sur l'extrémité distale (13) de la première lumière (3) et la première lumière (3) présente, sur le côté faisant face à l'orifice d'aspiration (5), un orifice de compensation de pression (10).

2. Cathéter d'aspiration selon la revendication 1, **caractérisé en ce que** le fil de stabilisation (11) se termine directement sur le côté distal de l'orifice latéral (7) de la deuxième lumière.

3. Cathéter d'aspiration selon la revendication 1 ou 2, **caractérisé en ce que** la deuxième lumière (6) dépasse de l'extrémité distale (13) de la première lumière (3) et réalise avec son orifice distal (8) l'extrémité distale (12) du cathéter d'aspiration (1).

4. Cathéter d'aspiration selon l'une quelconque des revendications 1 à 3 avec une extrémité distale (12), qui est réalisée en forme d'ergot et présente une section transversale extérieure qui correspond à la section transversale extérieure de la deuxième lumière (6).

5. Cathéter d'aspiration selon la revendication 5, **caractérisé en ce que** l'orifice d'aspiration (5) est disposé sur le côté faisant face à la deuxième lumière (6).

6. Cathéter d'aspiration selon la revendication 1, **caractérisé en ce que** l'orifice de compensation de pression (10) est décalé latéralement par rapport à la deuxième lumière (6).

7. Cathéter d'aspiration selon l'une quelconque des revendications précédentes, **caractérisé par** une bague de marquage (9) sur la deuxième lumière (6) à hauteur de l'orifice d'aspiration (5) de la première lumière (3).

8. Cathéter d'aspiration selon la revendication 7 avec une bague de marquage (9) composée d'un alliage de platine et d'iridium 90/10.

9. Cathéter d'aspiration selon l'une quelconque des revendications précédentes, **caractérisé en ce que** le fil de stabilisation (11) voit sa flexibilité augmenter depuis le côté proximal vers le côté distal.

10. Cathéter d'aspiration selon l'une quelconque des revendications précédentes, **caractérisé par** un revêtement hydrophile extérieur (17).

11. Cathéter d'aspiration selon la revendication 10, **caractérisé en ce que** le revêtement hydrophile (17) se termine sur le côté proximal par rapport à l'orifice latéral (7) de la deuxième lumière (6).

12. Cathéter d'aspiration selon l'une quelconque des revendications précédentes, **caractérisé en ce que** le fil de stabilisation (11) est pourvu dans la zone de son extrémité distale d'un marquage, qui indique, lors du retrait du fil de stabilisation (11) hors du cathéter (1), la longueur restante dans le cathéter (1).

13. Cathéter d'aspiration selon l'une quelconque des revendications précédentes, **caractérisé en ce que** la zone distale (2) de la première lumière (3) présente une section transversale ovale, dans lequel l'orifice d'aspiration (5) est disposé sur le côté de l'axe principal de l'ovale.

14. Cathéter d'aspiration selon l'une quelconque des revendications 1 à 13 avec un fil de guidage adapté à celui-ci en termes de dimension et un cathéter de guidage.

15. Ensemble de cathéters selon la revendication 14 avec au moins une seringue pour l'extraction par aspiration de sang contenant des thrombus.
